(19) Europäisches Patentamt
European Patent Office
Office européen des brevets

(11) Veröffentlichungsnummer: **0 346 724**
**A1**

(12) **EUROPÄISCHE PATENTANMELDUNG**

(21) Anmeldenummer: **89110177.6**

(22) Anmeldetag: **06.06.89**

(51) Int. Cl.⁴: **C07D 405/04 , C07D 405/12 , A61K 31/44 , //(C07D405/04, 311:00,211:00),(C07D405/04, 311:00,237:00),(C07D405/12, 311:00,211:00),(C07D405/12, 311:00,237:00)**

(30) Priorität: **16.06.88 DE 3820506**
**14.10.88 DE 3835011**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Häusler, Günther, Prof. Dr.**
**Albert-Schweitzer-Strasse 13**
**D-6104 Seeheim(DE)**
Erfinder: **Gericke, Rolf, Dr.**
**Mozartstrasse 19**
**D-6104 Seeheim(DE)**
Erfinder: **Wurziger, Hanns, Dr.**
**Greinstrasse 7B**
**D-6100 Darmstadt(DE)**
Erfinder: **Baumgarth, Manfred, Dr.**
**Sachsenstrasse 53**
**D-6100 Darmstadt(DE)**
Erfinder: **Lues, Inge, Dr.**
**Paul-Wagner-Strasse 13**
**D-6100 Darmstadt(DE)**
Erfinder: **Bergmann, Rolf, Dr.**
**Birkenhag 36**
**D-6101 Reichelsheim(DE)**
Erfinder: **De Peyer, Jacques, Dr.**
**Bleichweg 15**
**D-6104 Seeheim(DE)**

(54) **Chromanderivate.**

(57) Neue Chromanderivate der Formel I

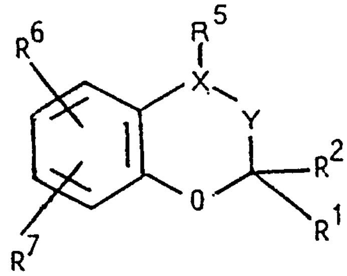

I

worin X, Y, $R^1$, $R^2$, $R^5$, $R^6$ und $R^7$ die in Patentanspruch 1 angegebenen Bedeutungen haben, sowie ihre Salze zeigen Wirkungen auf das cardiovaskuläre System und können verwendet werden zur Behandlung bzw. Prophylaxe von Herzinsuffizienz, Angina pectoris, Bluthochdruck, Inkontinenz und Alopezie.

EP 0 346 724 A1

## Chromanderivate

## Chromanderivate

Die Erfindung betrifft neue Chromanderivate der Formel I

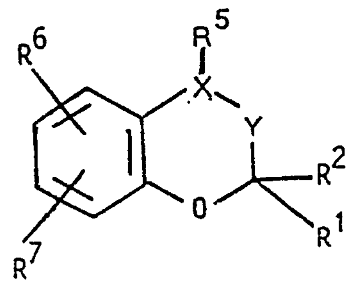

I

worin

X-Y

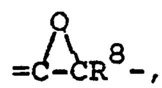

$=CH-CO-$ oder $=CH-CHR^8-$, sowie, falls der Rest $R^5$ weder vollständig noch partiell hydriert ist, auch $=CR^4-CR^3R^8-$oder $=CH-CR=^8(OA)-$,

$R^1$ A,

$R^2$ und $R^8$ jeweils H oder A,

$R^1$ und $R^2$ zusammen auch Alkylen mit 3-6 C-Atomen,

$R^3$ OH oder OAc,

$R^4$ H,

$R^3$ und $R^4$ zusammen auch eine Bindung,

$R^5$ einen unsubstituierten oder einen ein- oder zweifach durch A, F, Cl, Br, J, OH, OA, OAc, $NO_2$, $NH_2$ AcNH, HOOC und/oder AOOC substituierten Pyridyl-oxy-, Pyridazinyl-oxy-, Pyrimidinyl-oxy-, Pyrazinyl-oxy-, Oxo-dihydro-pyridyl-oxy-, Oxo-dihydro-pyridazinyl-oxy-, Oxo-dihydropyrimidinyl-oxy-, Oxo-dihydro-pyrazinyl-oxy-, 1H-2- oder 1H-4-Pyridon-1-yl-, 1H-4- oder 1H-6-Pyridazinon-1-yl-, 1H-2-, 1H-4- oder 1H-6-Pyrimidinon-1-yl-, 1H-2-Pyrazinon-1-yl-, 3H-oder 5H-2-Pyrrolinon-1-yl-, 1H-2-Chinolinon-1-yl-, 2H-1-Isochinolinon-2-yl-, 2H-1-Phthalazinon-2-yl-, 3H-4-Chinazolinon-3-yl- oder 1H-2-Thiopyridon-1-yl-rest, wobei diese Reste auch vollständig oder partiell hydriert sein können,

$R^6$ und $R^7$ jeweils H, A, HO, AO, CHO, ACO, ACS, HOOC, AOOC, AO-CS, ACOO, A-CS-0, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyan-alkyl, A-C(=NOH) oder A-C(=$NNH_2$),

$R^7$ auch Pyridyl, Pyridazinyl, Pyrimidinyl oder Pyrazinyl,

A Alkyl mit 1-6 C-Atomen,

alkyl Alkylen mit 1-6 C-Atomen und

Ac Alkanoyl mit 1-8 C-Atomen oder Aroyl mit 7-11 C-Atomen

bedeuten,

sowie deren Salze.

Insbesondere betrifft die Erfindung Verbindungen der obigen Formel I, worin

X-Y 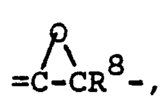

$=CH-CO-$ oder $=CH-CHR^8$, sowie, falls $R^5$ einen unsubstituierten oder einen ein- oder zweifach durch A, F, Cl, Br, J, OH, OA, OAc, $NO_2$, $NH_2$, AcNH, HOOC und/oder AOOC substituierten 1H-4-Pyridon-1-yl-, 1H-4-Pyridazinon-1-yl-, 1H-4-Pyrimidinon-1-yl-, 1H-2-Chinolinon-1-yl-, 2H-1-Isochinolinon-2-yl-, 2H-1-Phthalazinon-2-yl- oder 3H-4-Chinazolinon-3-yl-Rest (wobei diese Reste auch vollständig oder partiell hydriert sein können) und/oder falls $R^7$ Pyridyl, Pyridazinyl, Pyrimidinyl oder Pyrazinyl bedeutet, auch $=CR^4\text{-}CR^3R^8\text{-}$, oder, falls der Rest $R^5$ weder vollständig noch partiell hydriert ist, auch $=CH\text{-}CR^8(OA)\text{-}$ bedeutet.

Ähnliche Verbindungen sind bekannt aus der EP-A1-76075 und der EP-A1-173848.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z.B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z.B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls $R^1$ und $R^2$ zusammen Alkylen bedeuten, so ist die Alkylengruppe vorzugsweise unverzweigt, im einzelnen bevorzugt $-(CH_2)_n-$, wobei n 3, 4, 5 oder 6 bedeutet.

Die Gruppe "alkyl" steht vorzugsweise für $-CH_2-$ oder $-CH_2CH_2-$.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

Die Gruppe X-Y ist vorzugsweise $=CR^4\text{-}CR^3R^8\text{-}$, im einzelnen bevorzugt $=CH\text{-}CR^8(OH)\text{-}$ oder $=C=CR^8\text{-}$, insbesondere bevorzugt $=CH\text{-}CHOH\text{-}$ oder $=C=CH\text{-}$; sie bedeutet ferner bevorzugt

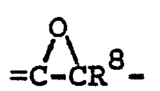 (insbesondere 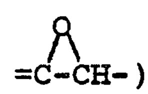),

$=CH\text{-}CO\text{-}$, $=CH\text{-}CHR^8\text{-}$(insbesonder $=CH\text{-}CH_2$) oder $=CH\text{-}CR^8(OA)\text{-}$ [insbesondere $=CH\text{-}CH(OA)\text{-}$].

$R^1$ und $R^2$ sind vorzugsweise jeweils Alkyl, insbesondere jeweils Methyl oder Ethyl, bevorzugt jeweils Methyl.

$R^3$ und $R^4$ sind bevorzugt zusammen eine Bindung. Falls $R^4$ H bedeutet, ist $R^3$ bevorzugt OH, O-CHO oder $O\text{-}COCH_3$.

$R^5$ ist bevorzugt unsubstituiertes 1H-2-Pyridon-1-yl, 2-Hydroxy-4-pyridyl-oxy oder 6-Hydroxy-3-pyridazinyl-oxy-, weiterhin 2-, 3- oder 4-Pyridyl-oxy, 3-Hydroxy-1H-6-pyridazinon-1-yl oder 1H-4-Hydroxy-2-pyridon-1-yl, ferner bevorzugt unsubstituiertes 1H-2-Pyrazinon-1-yl, 1H-6-Pyridazinon-1-yl, 4,5-Dihydro-1H-6-pyridazinon-1-yl, 1H-2-Pyrimidinon-1-yl, 1H-6-Pyrimidinon-1-yl, 3H- oder 5H-Pyrrolinon-1-yl oder 1H-2-Thiopyridon-1-yl. Weiterhin bedeutet $R^5$ bevorzugt unsubstituiertes 1H-4-Pyridon-1-yl, 1H-4-Pyridazinon-1-yl, 1H-4-Pyrimidinon-1-yl, 1H-2-Chinolinon-1-yl, 2H-1-Isochinolinon-2-yl, 2H-1-Phthalazinon-2-yl oder 3H-4-Chinazolinon-3-yl. Falls $R^5$ einen substituierten Pyridon- bzw. Thiopyridonring bedeutet, so ist dieser Ring vorzugsweise einfach in 3-, 4- oder 5-Stellung oder zweifach in 3- und 5-Stellung substituiert. Besonders bevorzugte Substituenten sind OH, $NO_2$ und $NH_2$ ferner AOOC, OA, Cl, Br und $NHCOCH_3$, besonders bevorzugte substituierte Reste $R^5$ im einzelnen 4-, ferner 3-, 5- und 6-Hydroxy-, 3-, 4-, 5- oder 6-Methoxy-, 3-, 4-, 5- oder 6-Acetoxy-, 3-, 5- oder 6-Chlor-, 3- oder 5-Nitro-, 3-oder 5-Amino-, 3- oder 5-Carboxy-, 3-oder 5-Methoxycarbonyl-, 3- oder 5-Ethoxycarbonyl-, 3- oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-, 3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido- und 3-Acetamido-5-brom-1H-2-pyridon-1-yl bzw. -1H-2-thiopyridon-1-yl, 1H-4- oder 1H-5-Hydroxy-6-pyridazinon-1-yl, 1H-3-, 1H-4- oder 1H-5-Methoxy-6-pyridazinon-1-yl, 1H-3-, 1H-4-oder 1H-5-Ethoxycarbonyl-6-pyridazinon-1-yl, 1H-4-, 1H-5- oder 1H-6-Hydroxy-2-pyrimidinon-1-yl, 1H-2- oder 1H-4-Hydroxy-6-pyrimidinon-1-yl.

$R^5$ kann ferner bevorzugt bedeuten: 3,4-Dihydro-1H-2-pyridon-1-yl, 2,3-Dihydro-6H-2-pyridon-1-yl, 5,6-Dihydro-1H2-pyridon-1-yl, 2-Piperidinon-1-yl, 2,3-Dihydro-1H-6-pyridazinon-1-yl, 1,2-Dihydro-5H-6-pyridazinon-1-yl, 4,5-Dihydro-1H-6-pyridazinon-1-yl, 2,3,4,5-Tetrahydro-1H-6-pyridazinon-1-yl, 3,4-Dihydro-1H-2-pyrimidinon-1-yl, 1,6-Dihydro-3H-2-pyrimidinon-1-yl, 5,6-Dihydro-1H-2-pyrimidinon-1-yl, 3,4,5,6-Tetrahydro-1H-2-pyrimidinon-1-yl, 2,3-Dihydro-1H-6-pyrimidinon-1-yl, 1,2-Dihydro-5H-6-pyrimidinon-1-yl, 4,5-Dihydro-1H-6-pyrimidinon-1-yl, 2,3,4,5-Tetrahydro-1H-6-pyrimidinon-1-yl, 3,4-Dihydro-1H-2-pyrazinon-1-yl, 1,6-Dihydro-3H-2-pyrazinon-1-yl, 5,6-Dihydro-1H-2-pyrazinon-1-yl, 3,4,5,6-Tetrahydro-1H-2-pyrazinon-1-yl-2-Pyrrolidinon-1-yl, 3,4-Dihydro-1H-2-thiopyridon-1-yl, 2,3-Dihydro-6H-2-thiopyridon-1-yl, 5,6-Dihydro-1H-2-thiopyridon-1-yl.

In $R^6$ und $R^7$ bedeuten vorzugsweise:

| | |
|---|---|
| A: | Methyl, ferner Ethyl; |
| AO: | Methoxy, ferner Ethoxy; |
| ACO: | Acetyl, ferner Propionyl; |
| ACS: | Thioacetyl, ferner Thiopropionyl; |
| AOOC: | Methoxycarbonyl, ferner Ethoxycarbonyl; |
| AO-CS: | Methoxy-thiocarbonyl, ferner Ethoxy-thiocarbonyl; |
| ACOO: | Acetoxy, ferner Propionoxy; |
| ACSO: | Thio(no)acetoxy, ferner Thio(no)propionoxy; |
| Hydroxyalkyl: | Hydroxymethyl oder 1- oder 2-Hydroxyethyl; |
| Mercaptoalkyl: | Mercaptomethyl oder 1- oder 2-Mercapto-ethyl; |
| NHA: | Methylamino, ferner Ethylamino; |
| $NA_2$: | Dimethylamino, ferner Diethylamino; |
| ASO: | Methylsulfinyl, ferner Ethylsulfinyl; |
| $ASO_2$: | Methylsulfonyl, ferner Ethylsulfonyl; |
| AO-SO: | Methoxy-sulfinyl, ferner Ethoxy-sulfinyl; |
| $AO-SO_2$: | Methoxy-sulfonyl, ferner Ethoxy-sulfonyl; |
| Ac-NH: | Acetamido, ferner Formamido, Propionamido oder Benzamido; |
| AO-CO-NH: | Methoxycarbonylamino, ferner Ethoxycarbonylamino; |
| HANSO: | Methylaminosulfinyl, ferner Ethylamino-sulfinyl |
| $A_2NSO$: | Dimethylaminosulfinyl, ferner Diethyl-aminosulfinyl; |
| $HANSO_2$: | Methylaminosulfonyl, ferner Ethylamino-sulfonyl; |
| $A_2NSO_2$: | Dimethylaminosulfonyl, ferner Diethyl-aminosulfonyl; |
| HANCO: | N-Methylcarbamoyl, ferner N-Ethylcarbamoyl; |
| $A_2NOC$: | N,N-Dimethylcarbamoyl, ferner N,N-Diethyl-carbamoyl; |

| | |
|---|---|
| HANCS: | N-Methyl-thiocarbamoyl, ferner N-Ethyl-thiocarbamoyl; |
| $A_2NCS$: | N,N-Dimethyl-thiocarbamoyl, ferner N,N-Diethyl-thiocarbamoyl; |
| ASONH: | Methylsulfinylamino, ferner Ethylsulfinyl-amino; |
| $ASO_2NH$: | Methylsulfonylamino, ferner Ethylsulfonyl-amino; |
| AOSONH: | Methoxysulfinylamino, ferner Ethoxy-sulfinylamino; |
| $AOSO_2NH$: | Methoxysulfonylamino, ferner Ethoxy-sulfonylamino; |
| ACO-alkyl: | 2-Oxopropyl, 2-Oxobutyl, 3-Oxobutyl, 3-Oxopentyl; |
| Nitroalkyl: | Nitromethyl, 1- oder 2-Nitroethyl; |
| Cyanalkyl: | Cyanmethyl, 1- oder 2-Cyanethyl; |
| A-C(= NOH): | 1-Oximinoethyl, ferner 1-Oximinopropyl; |
| $A-C(=NNH_2)$: | 1-Hydrazonoethyl, ferner 1-Hydrazonopropyl. |

Der Rest $R^7$ kann ferner 2-, 3- oder (bevorzugt) 4-Pyridyl bedeuten, weiterhin 3- oder 4-Pyridazinyl, 2-, 4-oder 5-Pyrimidinyl oder Pyrazinyl.

Die Reste $R^6$ und $R^7$ stehen vorzugsweise in 6- und 7-Stellung des Chromansystems. Sie können jedoch auch in 5- und 6-, 5- und 7-, 5- und 8-, 6- und 8- sowie 7- und 8-Stellung stehen.

Von den Resten $R^6$ und $R^7$ ist vorzugsweise der eine H, während der andere von H verschieden ist. Dieser andere Rest steht vorzugsweise in 6-Stellung, aber auch in 5-, 7- oder 8-Stellung, und ist vorzugsweise CN oder $NO_2$, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, $CF_3$, $H_2NCO$, $H_2NCS$, $NH_2$ oder 4-Pyridyl.

Der Rest $R^8$ ist vorzugsweise H, weiterhin bevorzugt Methyl oder Ethyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ii ausgedrückt werden, die der Formel I entsprechen und worin die nicht näher bezeichneten Reste die bei der Formel I angegebene Bedeutung haben, worin jedoch

| | | |
|---|---|---|
| in Ia | $R^1$ und $R^2$ jeweils A bedeuten; | |
| in Ib | $R^1$ und $R^2$ jeweils $CH_3$ bedeuten; | |
| in Ic | $R^1$ und $R^2$ zusammen Alkylen mit 3-6 C-Atomen bedeuten; | |
| in Id | $R^5$ | 1H-2-Pyridon-1-yl, 2-Pyrrolidinon-1-yl, 2-Hydroxy-4-pyridyl-oxy oder 6-Hydroxy-3-pyridazinyl-oxy bedeutet; |
| in Ie | $R^5$ | 1H-2-Pyridon-1-yl, 2-Hydroxy-4-pyridyl-oxy oder 6-Hydroxy-3-pyridazinyl-oxy bedeutet; |
| In If | $R^5$ | 1H-2-Pyridon-1-yl bedeutet; |
| in Ig | $R^1$ und $R^2$ jeweils $CH_3$ und | |
| | $R^5$ | 1H-2-Pyridon-1-yl, 2-Pyrrolidinon-1-yl, 2-Hydroxy-4-pyridyl-oxy oder 6-Hydroxy-3-pyridazinyl-oxy bedeutet; |
| in Ih | $R^1$ und $R^2$ jeweils $CH_3$ und | |
| | $R^5$ | 1H-2-Pyridon-1-yl, 2-Pyrrolidinon-1-yl, 2-Hydroxy-4-pyridyl-oxy oder 6-Hydroxy-3-pyridazinyl-oxy bedeuten; |
| in Ii | $R^1$ und $R^2$ jeweils $CH_3$ und | |
| | $R^5$ | 1H-2-Pyridon-1-yl bedeuten. |

Weiterhin sind bevorzugt Verbindungen der Formeln I′ sowie Ia′ bis Ii′, die den Formeln I sowie Ia bis Ii entsprechen, worin jedoch jeweils zusätzlich -X-Y- =C-CR$^8$(OH)-bedeutet.

Weiterhin sind bevorzugt Verbindungen der Formeln I″ sowie Ia″ bis Ii″, die den Formeln I sowie Ia bis Ii entsprechen, worin jedoch jeweils zusätzlich -X-Y- =C=CR⁸-bedeutet.

Ferner sind bevorzugt Verbindungen der Formeln I, I′, I, Ia bis Ii, Ia′ bis Ii′ sowie Ia″ bis Ii, worin jeweils zusätzlich

(a)
$R^6$ von H verschieden ist und
$R^7$ H bedeutet;

(b)
$R^6$ von H verschieden ist und in 6-Stellung steht und
$R^7$ H bedeutet;

(c)
$R^6$ $NO_2$, CN, CHO, ACO, HOOC, AOOC, ACOO, F, Cl, Br, J, $CF_3$, $H_2NCO$, $H_2NCS$ oder $NH_2$ und
$R^7$ H bedeutet;

(d)
$R^6$ $NO_2$, CN, CHO, ACO, HOOC, AOOC, ACOO, F, Cl, Br, J, $CF_3$, $H_2NCO$, $H_2NCS$ oder $NH_2$ bedeutet und in 6-Stellung steht und
$R^7$ H bedeutet;

(e)
$R^6$ $NO_2$, CN, CHO, $CH_3CO$, $CH_3OOC$,$C_2H_5OOC$ oder $CH_3COO$ und
$R^7$ H bedeutet;

(f)
$R^6$ $NO_2$, CN, CHO, $CH_3CO$, $CH_3OOC$,$C_2H_5OOC$ oder $CH_3COO$ bedeutet und in 6-Stellung steht und
$R^7$ H bedeutet;

(g)
$R^6$ $NO_2$ oder CN und
$R^7$ H bedeutet;

(h)
$R^6$ $NO_2$ oder CN bedeutet und in 6-Stellung steht und
$R^7$ H bedeutet;

(i)
$R^6$ CN und
$R^7$ H bedeutet;

(j)
$R^6$ CN bedeutet und in 6-Stellung steht und
$R^7$ H bedeutet.

Insbesondere sind bevorzugt Verbindungen der Formeln I, I′, I″, Ia bis Ii, Ia′ bis Ii′, Ia″ bis Ii″ sowie der übrigen vorstehend als bevorzugt gekennzeichneten Gruppen von Verbindungen, worin zusätzlich $R^8$ H bedeutet. Im übrigen haben vor- und nachstehend die Reste $R^1$ bis $R^8$, A, "alkyl" und Ac die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I, dadurch gekennzeichnet, daß man zur Herstellung von Verbindungen der Formel I, worin X-Y =$CR^4$-$CR^3R^8$ oder =CH-$CHR^8$- bedeutet, ein 3,4-Epoxychroman der Formel IIa oder ein Chromanderivat der Formel IIb

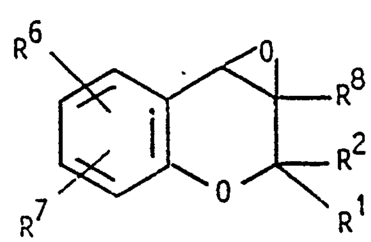

IIa

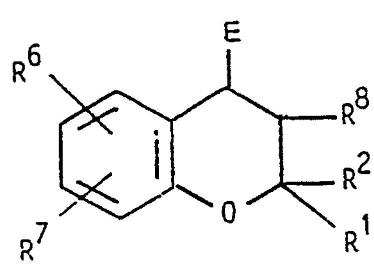

IIb

worin

E Cl, Br, J oder eine reaktionsfähig veresterte OH-Gruppe bedeutet und
$R^1$, $R^2$, $R^6$, $R^7$ und $R^8$ die bei Formel I angegebene Bedeutung haben,
mit einer Verbindung der Formel III

$R^5$-H III

worin $R^5$ die bei Formel I angegebene Bedeutung hat oder mit einem ihrer reaktionsfähigen Derivate umsetzt
und/oder daß man eine Verbindung der Formel I, worin X-Y = CH-$CR^8$(OH)- bedeutet, dehydratisiert oder alkyliert und/oder daß man eine Verbindung der Formel I, worin X-Y = C = $CR^8$- bedeutet, epoxidiert und/oder daß man eine Verbindung der Formel I, worin

X-Y 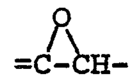

bedeutet, durch Behandeln mit einem sauren Katalysator zu einer Verbindung der Formel I, worin X-Y = CH-CO- bedeutet, isomerisiert und/oder daß man in einer Verbindung der Formel I einen oder mehrere der Reste $R^3$, $R^5$, $R^6$ und /oder $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel IIa oder IIb mit Verbindungen der Formel III hergestellt, zweckmäßig in Gegenwart eines inerten Lösungsmittels bei Temperaturen zwischen etwa 0 und 150°.

Die Ausgangsstoffe IIa, IIb und III sind in der Regel bekannt. Sofern sie nicht bekannt sind, können sie nach an sich bekannten Methoden hergestellt werden. So sind die Ausgangsstoffe der Formel IIa erhältlich durch Umsetzung von 2-Hydroxyacetophenonen der Formel 2-HO-$R^6R^7C_6H_2$-$COCH_3$ mit Ketonen der Formel $R^1$-CO-$R^2$ zu entsprechenden 4-Chromanonen der Formel IVa

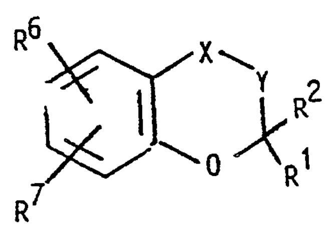

IVa -X-Y- = -CO-$CH_2$-
IVb -X-Y- = -CO-C(=CH-$R^9$)-
IVc -X-Y- = -CHOH-$CHR^8$-
IVd -X-Y- = -CH=$CR^8$-
IVe -X-Y- = -CHBr-$CR^8$(OH)-

gegebenenfalls Kondensation mit Aldehyden Formel $R^9$-CHO ($R^9$ = Alkyl mit 1-5 C-Atomen) zu 3-Alkyliden-4-chromanonen der Formel IVb, Reduktion, z.B. mit $NaBH_4$, zu 4-Chromanolen der Formel IVc, Dehydratisierung, z.B. mit p-Toluolsulfonsäure, zu Chromenen der Formel IVd und Oxydation, z.B. mit 3-Chlorperbenzoesäure. Die letztgenannte Oxydation kann auch mehrstufig erfolgen. So kann man, z.B. mit N-Bromsuccinimid in wäßriger Lösung zunächst die Bromhydrine der Formel Ve herstellen und aus diesen anschließend mit einer Base, z.B. Natronlauge, HBr abspalten.

Man kann die Chromene der Formel IVd auch erhalten durch Kondensation von Salicylaldehyden der Formel 2-HO-$R^6R^7$ $C_6H_2$-CHO mit Ketonen der Formel $R^1$-CO-$CH_2$-$R^8$ zu Hydroxyketonen der Formel 2-HO-$R^6R^7C_6H_2$-CH = $CR^8$-CO-$R^1$, Umsetzung mit Organo-Li-Verbindungen der Formel $R^2$-Li und nachfolgende Hydrolyse zu Diolen der Formel 2-HO-$R^6R^7C_6H_2$-CH = $CR^8$-$CR^1R^2$-OH und Cyclisierung unter Wasserabspaltung.

Ausgangsstoffe der Formel IIa ($R^8$ = H) sind ferner erhältlich durch Umsetzung von Propargylchloriden der Formel HC≡C-$CR^1R^2$-Cl mit Phenolen der Formel $R^6R^7C_6H_3OH$ zu Phenolethern der Formel $R^6$ $R^7C_6H_3O$-$CR^1R^2$-C≡CH, Cyclisierung zu 2H-Chromenen entsprechend Formel I, worin aber an Stelle der

Gruppe -$XR^5$-Y- die Gruppe -CH=CH- steht, Anlagerung von HOBr zum Bromhydrin der Formel IVe ($R^8$=H) und Dehydrobromierung.

In den Verbindungen der Formel IIb kommen als "reaktionsfähig veresterte OH-Gruppen" insbesondere die Ester mit Alkylsulfonsäuren (worin die Alkylgruppe 1-6 C-Atome enthält) oder mit Arylsulfonsäuren (worin die Arylgruppe 6-10 C-Atome enthält) in Betracht.

Verbindungen der Formel IIb sind erhältlich aus den 4-Chromanolen der Formel IVc durch Umsetzung mit einem anorganischen Säurehalogenid wie $PCl_3$, $PBr_3$, $SOCl_2$ oder $SOBr_2$ oder mit einem Sulfonsäurechlorid wie Methan- oder p-Toluolsulfonsäurechlorid.

Als reaktionsfähige Derivate von III eignen sich die entsprechenden Salze, z.B. die Na- oder K-Salze, die auch in situ entstehen können.

Bei der Umsetzung von II mit III können verschiedene Produkte der Formel I entstehen, abhängig insbesondere von der Struktur der Ausgangsstoffe III und von den Reaktionsbedingungen.

Beispielsweise ist die Bildung von Verbindungen der Formel I mit einer Sauerstoffbrücke ($R^5$ = gegebenenfalls substituiertes Pyridyl-oxy, Pyridazinyl-oxy, Pyrimidinyloxy, Pyrazinyl-oxy, Oxo-dihydro-pyridyl-oxy, Oxo-dihydro-pyridazinyl-oxy, Oxo-dihydro-pyrimidinyl-oxy oder Oxo- dihydro-pyrazinyl-oxy) begünstigt, wenn die Verbindung III zusätzlich zu der Lactam- bzw. Lactimgruppierung mindestens eine OH-Gruppe als Substituenten enthält und/oder wenn man unter relativ milden Bedingungen, z.B. in Gegenwart einer schwachen Base wie Pyridin in einem Alkohol, arbeitet. Beispielsweise entstehen aus 2,2-Dimethyl-3,4-epoxy-6-cyan-chroman ("IIc") und 1H-2-Pyridon mit NaH in DMSO überwiegend 2,2-Dimethyl-4-(1H-2-pyridon-1-yl)-6-cyan-2H-chromen ("A") und 2,2-Dimethyl-4-(1H-2-pyridon-1-yl)-6-cyan-3-chromanol ("B"), mit Pyridin in Ethanol dagegen "B" neben wenig 2,2-Dimethyl-4-(2-pyridyl-oxy)-6-cyan-3-chromanol. Aus 2,4-Dihydroxypyridin (= 4-Hydroxy-1H-2-pyridon) und IIc entsteht in Pyridin/Ethanol 2,2-Dimethyl-4-(2-hydroxy-4-pyridyl-oxy)-6-cyan-3-chromanol neben wenig 2,2-Dimethyl-4-(4-hydroxy-1H-2-pyridon-1-yl)-6-cyan-3-chromanol.

Eine Optimierung der Reaktionsbedingungen gelingt in jedem Einzelfall leicht. Die Reaktionsprodukte können ohne Schwierigkeiten getrennt und isoliert werden, z.B. durch Kristallisation und/oder Chromatographie.

Es ist zweckmäßig, in Gegenwart einer Base zu arbeiten. Als Basen eignen sich z.B. Alkalimetall- oder Erdalkalimetall-hydroxide, -carbonate, -alkoholate, -hydride oder auch -amide wie NaOH, KOH, $Ca(OH)_2$, $Na_2CO_3$, $K_2CO_3$, Na-oder K-methylat, -ethylat oder -tert.-buylat, NaH, KH, $CaH_2$, $NaNH_2$, $KNH_2$, ferner organische Basen wie Triethylamin oder Pyridin, die auch im Überschuß angewendet werden können und dann gleichzeitig als Lösungsmittel dienen.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl- oder -monoethylether (Methylglykol oder Ethylgly kol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II kann auch in situ hergestellt werden, z.B. durch Einwirkung einer Base auf das entsprechende Bromhydrin Ve.

Eine Verbindung der Formel I, worin -X-Y- =CH-$CR^8$(OH)-bedeutet, kann durch Behandeln mit einem Dehydratisierungsmittel in eine Verbindung der Formel I, worin -X-Y-=CH=$CR^8$- bedeutet, umgewandelt werden. Das gelingt z.B. durch Einwirkung einer der angegebenen Basen, z.B. NaOH, KOH oder NaH, in einem der angegebenen Lösungsmittel, z.B. Tetrahydrofuran, Dioxan oder DMSO, bei Temperaturen zwischen 0 und 150°.

Ferner kann eine Verbindung der Formel I, worin -X-Y-=CH-$CR^8$(OH)- bedeutet, alkyliert werden, wobei eine 3-Alkoxyverbindung der Formel I, worin -X-Y- =CH-$CR^8$(OA)-bedeutet gebildet wird. Man alkyliert vorzügsweise mit entsprechenden Alkylhalogeniden oder -sulfonaten der Formel A-E, z.B. A-Cl, A-Br, A-J, A-$OSO_2$A, A-$OSO_2$Aryl wie Methylchorid, -bromid, -jodid, -methansulfonat, -benzolsulfonat, oder auch mit Dialkylsulfaten der Formel $A_2SO_4$, z.B. Dimethylsulfat, Diethylsulfat, zweckmäßig in Gegenwart eines der angegebenen inerten Lösungsmittel wie Toluol oder DMF und/oder in Gegenwart einer der an gegebenen Basen wie $K_2CO_3$ oder K-tert.-Butylat bei Temperaturen zwischen 0 und 120°. Falls gleichzeitig weitere OH-Gruppen im Molekül vorhanden sind, so können diese gleichzeitig ebenfalls alkyliert werden.

Verbindungen der Formel I, worin -X-Y- =C=$CR^8$- bedeutet, können epoxidiert werden, wobei Epoxide der Formel I entstehen, worin

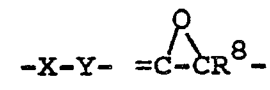

bedeutet. Man epoxidiert zweckmäßig mit einem Derivat des Wasserstoffperoxids, vorzugsweise einer Persäure wie Perbenzoesäure oder 3-Chlorperbenzoesäure in einem inerten Lösungsmittel wie Dichlormethan bei Temperaturen zwischen 0 und 60°.

Epoxide der Formel I, worin

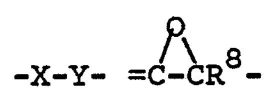

bedeutet, können durch Behandeln mit sauren Katalysatoren, zweckmäßig mit Mineralsäuren wie HCl, HBr oder $H_2SO_4$ in inerten Lösungsmitteln wie Dioxan bei Temperaturen zwischen 0 und 120°, in die isomeren Ketone der Formel I, worin -X-Y- =CH-CO- bedeutet, umgewandelt werden.

Weiterhin kann man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandeln.

Beispielsweise ist es möglich, daß man ein H-Atom mittels einer Halogenierung durch ein Halogenatom oder einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z.B. mit Raney-Nickel in Wasser/Essigsäure/Pyridin in Gegenwart von Natriumphosphat) in eine Formylgruppe oder (z.B. mit KOH in tert.-Butanol) in eine Carbamoylgruppe oder (z.B. mit $H_2S$ in Pyridin/Triethylamin) in eine Thiocarbamoylgruppe umwandelt und/oder einen substituierten oder unsubstituierten 1H-2-Pyridon-1-ylrest (z.B. mit $P_2S_5$ oder mit Lawesson-Reagenz in Toluol) in den entsprechenden 1H-2-Thiopyridon-1-ylrest umwandelt.

Eine Nitrierung gelingt unter üblichen Bedingungen, z.B. mit einem Gemisch aus konzentrierter $HNO_3$ und konzentrierter $H_2SO_4$ bei Temperaturen zwischen 0 und 30°. Falls mindestens einer der Substituenten $R^6$ und $R^7$ eine elektronegative Gruppe wie CN oder $NO_2$ bedeutet, erfolgt die Nitrierung überwiegend am Rest $R^5$; andernfalls erhält man in der Regel Gemische, bei denen die Nitrogruppen am Rest $R^5$ oder am Chromanring stehen können.

Analoges gilt für die Halogenierung, die z.B. mit elementarem Chlor oder Brom in einem der üblichen inerten Lösungsmittel bei Temperaturen zwischen etwa 0 und 30° durchgeführt werden kann.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z.B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z.B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungemittel, z.B. DMF, bei Temperaturen zwischen etwa 0° und etwa 120° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z.B. Chloride oder Bromide) oder Anhydride von Carbonsäuren der Formel Ac-OH, z.B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z.B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0° und etwa 160°, vorzugsweise zwischen 20° und 120°. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-

oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z.B. Verbindungen der Formel I, worin $R^1 = R^2$, $R^3$ = OH und $R^4$ = H ist, zwei chirale Zentren; bei der Herstellung durch Reaktion von IIa mit III entsteht jedoch ganz überwiegend nur ein Racemat mit trans-Stellung der Substituenten $R^3$ = OH und $R^5$. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Carbinole (I, $R^3$ = OH) können ferner mit Hilfe chiraler Acylierungsreagenzien, z.B. D- oder L-α-Methylbenzylisocyanat, verestert und dann getrennt werden (vgl. EP-A1-120428). Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. in Alkoholen wie Ethanol oder Isopropanol, Acetonitril, DMF, Dimethylacetamid, 1,2-Propandiol oder deren Gemischen untereinander oder mit Wasser) oder Puder. Die neuen Verbindungen können auch lyophilisiert und die erhaltenen Lyophilisate z.B. zur Herstellung von Injektionspräparten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks-und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bwz. Blutdrucksenkern, z.B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung

liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopezie einschließlich der androgenen Alopezie und der Alopecia areata. Hierfür werden insbesondere pharmazeutische Zubereitungen verwendet, die zur topischen Behandlung der Kopfhaut geeignet und die oben genannt sind. Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation. Vor- und nachstehend sind alle Temperaturen in °C angegeben.

Beispiel 1

Ein Gemisch von 21,5 g 2,2,3-Trimethyl-3,4-epoxy-6-cyanchroman, 9,5 g 1H-2-Pyridon ("Pyridon"), 3 g einer 80%igen Dispersion von NaH in Paraffinöl und 600 ml DMSO wird 16 Std. bei 20° gerührt, in Wasser gegossen und mit Ethylacetat extrahiert. Man dampft ein und chromatographiert den Rückstand über Kieselgel. Mit Dichlormethan wird 2,2,3-Trimethyl-4-(1H-2-pyridon-1-yl)-6-cyan-2H-chromen (F. 212°) eluiert, anschließend mit Ethylacetat 2,2,3-Trimethyl-4-(1H-2-pyridon-1-yl)-6-cyan-chroman-3-ol (F. 185-186°).

Herstellung des Ausgangsmaterials:

(a) Ein Gemisch von 81 g 3-Acetyl-4-hydroxybenzonitril, 48 ml Aceton, 11,8 ml Pyrrolidin und 300 ml Toluol wird 1 Std. bei 20° stehengelassen, dann 2 Std. am Wasserabscheider gekocht und abgekühlt. Nach üblicher Aufarbeitung erhält man 2,2-Dimethyl-6-cyan-4-chromanon, F. 119-120°.

(b) Eine Lösung von 24 g des Chromanons, 12 g Paraformaldehyd und 24 ml Piperidin in 300 ml Ethanol wird 3 Std. auf 70° erhitzt und eingedampft. Man nimmt den Rückstand in Dichlormethan/Petrolether 1:1 auf, filtriert über Kieselgel, dampft ein und erhält 2,2-Dimethyl-3-methylen-6-cyan-4-chromanon als instabiles Öl.

(c) Eine Lösung von 25 g des vorstehenden Chromanons in 500 ml Methanol wird mit 6 g $NaBH_4$ versetzt, 1 Std. bei 20° gerührt und eingedampft. Nach üblicher Aufarbeitung erhält man 2,2,3-Trimethyl-6-cyan-4-chromanol, Isomerengemisch, ölig.

(d) Eine Lösung von 27 g des vorstehenden Gemischs und 1,2 g p-Toluolsulfonsäure in 400 ml Toluol wird 3 Std. am Wasserabscheider gekocht. Man dampft ein, löst in Dichlormethan/Petrolether 1:1, filtriert über Kieselgel, dampft erneut ein und erhält 2,2,3-Trimethyl-6-cyan-2H-chromen, F. 55°.

(e) Eine Lösung von 6,4 g m-Chlorperbenzoesäure in 40 ml Dichlormethan wird unter Rühren zu einer Lösung von 6,8 g des vorstehenden Chromens in 100 ml Dichlormethan zugetropft. Man rührt noch 16 Std., filtriert, gibt verdünnte Natronlauge zu, arbeitet wie üblich auf und erhält 2,2,3-Trimethyl-3,4-epoxy-6-cyan-chroman, F. 118°.

Analog erhält man

2,2-Dimethyl-4-(1H-2-pyridon-1-yl)-6-cyan-2H-chromen ("A"), F. 146-148°
2,2-Dimethyl-4-(1H-2-pyridon-1-yl)-6-cyan-3-chromanol ("B"), F. 224°
2,2-Dimethyl-4-(1H-5-chlor-2-pyridon-1-yl)-6-cyan-2H-chromen, F. 185-188°
2,2-Dimethyl-4-(1H-5-chlor-2-pyridon-1-yl)-6-cyan-3-chromanol, F. 268-270°
2,2-Dimethyl-4-(1H-3-hydroxy-2-pyridon-1-yl)-6-cyan-2H-chromen
2,2-Dimethyl-4-(1H-3-hydroxy-2-pyridon-1-yl)-6-cyan-3-chromanol, F. 262-265°
2,2-Dimethyl-4-(1H-4-hydroxy-2-pyridon-1-yl)-6-cyan-2H-chromen
2,2-Dimethyl-4-(1H-4-hydroxy-2-pyridon-1-yl)-6-cyan-3-chromanol, F. 256-257°
2,2-Dimethyl-4-(1H-5-hydroxy-2-pyridon-1-yl)-6-cyan-2H-chromen
2,2-Dimethyl-4-(1H-5-hydroxy-2-pyridon-1-yl)-6-cyan-3-chromanol, F. 256,5-258°
2,2-Dimethyl-4-(1H-3-methoxy-2-pyridon-1-yl)-6-cyan-2H-chromen
2,2-Dimethyl-4-(1H-3-methoxy-2-pyridon-1-yl)-6-cyan-3-chromanol,, F. 245-248°
2,2-Dimethyl-4-(1H-3-acetoxy-2-pyridon-1-yl)-6-cyan-2H-chromen

2,2-Dimethyl-4-(1H-3-acetoxy-2-pyridon-1-yl)-6-cyan-3-chromanol, F. 260-263°
2,2-Dimethyl-4-(1H-3-nitro-2-pyridon-1-yl)-6-cyan-2H-chromen, F. 148-150°
2,2-Dimethyl-4-(1H-3-nitro-2-pyridon-1-yl)-6-cyan-3-chromanol, F. 240-242°
2,2-Dimethyl-4-(1H-5-nitro-2-pyridon-1-yl)-6-cyan-2H-chromen, F. 214-216°
2,2-Dimethyl-4-(1H-5-nitro-2-pyridon-1-yl)-6-cyan-3-chromanol, F. 249-251°
2,2-Dimethyl-4-(1H-3-amino-2-pyridon-1-yl)-6-cyan-2H-chromen
2,2-Dimethyl-4-(1H-3-amino-2-pyridon-1-yl)-6-cyan-3-chromanol, F. 213-215°
2,2-Dimethyl-4-(1H-5-amino-2-pyridon-1-yl)-6-cyan-2H-chromen, F. 180°
2,2-Dimethyl-4-(1H-5-amino-2-pyridon-1-yl)-6-cyan-3-chromanol, F. 260-264°
2,2-Dimethyl-4-(1H-2-pyridon-1-yl)-6-nitro-2H-chromen, F. 158°
2,2-Dimethyl-4-(1H-2-pyridon-1-yl)-6-nitro-3-chromanol, F. 229-231°
2,2-Dimethyl-4-(1H-2-pyridon-1-yl)-6-acetyl-2H-chromen, F. 148-150°
2,2-Dimethyl-4-(1H-2-pyridon-1-yl)-6-acetyl-3-chromanol, F. 257-269°
2,2-Dimethyl-4-(1H-2-pyridon-1-yl)-6-methoxycarbonyl-2H-chromen, F. 126-127°
2,2-Dimethyl-4-(1H-2-pyridon-1-yl)-6-methoxycarbonyl-3-chromanol, F. 267-267,5°
2,2-Dimethyl-4-(1H-2-pyridon-1-yl)-6-ethoxycarbonyl-2H-chromen,
2,2-Dimethyl-4-(1H-2-pyridon-1-yl)-6-ethoxycarbonyl-3-chromanol, F. 213°
2,2-Tetramethylen-4-(1H-2-pyridon-1-yl)-6-cyan-2H-chromen, F. 181-183°
2,2-Tetramethylen-4-(1H-2-pyridon-1-yl)-6-cyan-3-chromanol, F. 227-230°
2,2-Pentamethylen-4-(1H-2-pyridon-1-yl)-6-cyan-2H-chromen, F. 202-204°
2,2-Pentamethylen-4-(1H-2-pyridon-1-yl)-6-cyan-2H-chromanol, F. 240-242°
2,2-Dimethyl-4-(1H-2-pyridazinon-1-yl)-6-cyan-2H-chromen, F. 136-138°
2,2-Dimethyl-4-(1H-2-pyridazinon-1-yl)-6-cyan-3-chromanol, F. 216-218°
2,2-Dimethyl-4-(4,5-dihydro-1H-6-pyridazinon-1-yl)-6-cyan-2H-chromen
2,2-Dimethyl-4-(4,5-dihydro-1H-6-pyridazinon-1-yl)-6-cyan-3-chromanol, F. 163-164,5°
2,2-Dimethyl-4-(1H-3-ethoxycarbonyl-6-pyridazinon-1-yl)-6-cyan-2H-chromen
2,2-Dimethyl-4-(1H-3-ethoxycarbonyl-6-pyridazinon-1-yl)-6-cyan-3-chromanol, F. 259-260,5°
2,2-Dimethyl-4-(1H-2-pyrimidinon-1-yl)-6-cyan-2H-chromen
2,2-Dimethyl-4-(1H-2-pyrimidinon-1-yl)-6-cyan-3-chromanol, F. 250-252°
2,2-Dimethyl-4-(1H-4-hydroxy-2-pyrimidinon-1-yl)-6-cyan-2H-chromen, F.278-279,5°
2,2-Dimethyl-4-(1H-4-hydroxy-2-pyrimidinon-1-yl)-6-cyan-3-chromanol, kein F. bis 300°
2,2-Dimethyl-4-(1H-6-pyrimidinon-1-yl)-6-cyan-2Hchromen
2,2-Dimethyl-4-(1H-6-pyrimidinon-1-yl)-6-cyan-3-chromanol, F. 207-208°
2,2,3-Trimethyl-4-(1H-2-pyridon-1-yl)-6-cyan-2H-chromen, F. 200°
2,2,3-Trimethyl-4-(1H-2-pyridon-1-yl)-6-cyan-3-chromanol
2,2,3-Trimethyl-4-(2-pyrrolidon-1-yl)-6-cyan-2H-chromen, F. 186°
2,2,3-Trimethyl-4-(2-pyrrolidon-1-yl)-6-cyan-3-chromanol
2,2-Dimethyl-4-(2-pyrrolidon-1-yl)-6-(4-pyridyl)-2H-chromen
2,2-Dimethyl-4-(2-pyrrolidon-1-yl)-6-(4-pyridyl)-3-chromanol, F. 238-239°
2,2-Dimethyl-3-ethyl-4-(1H-2-pyridon-1-yl)-6-cyan-2-H-chromen
2,2-Dimethyl-3-ethyl-4-(1H-2-pyridon-1-yl)-6-cyan-3-chromanol.

Beispiel 2

Ein Gemisch von 20,1 g IIc, 11,2 g 3,6-Pyridazindiol, 12 ml Pyridin und 600 ml Ethanol wird 72 Std. gekocht. Man destilliert etwa 300 ml ab, kühlt ab, filtriert nicht umgesetztes 3,6-Pyridazindiol ab und dampft ein. Das erhaltene 2,2-Dimethyl-4-(6-hydroxy-3-pyridazinyloxy)-6-cyan-3-chromanol wird aus Isopropanol umkristallisiert. F. 255-256°. Daneben entsteht wenig 2,2-Dimethyl-4-(3-hydroxy-1H-6-pyridazinon-1-yl)-6-cyan-3-chromanol, F. 260-262°.

Analog erhält man mit den entsprechenden Verbindungen der Formel $R^5$-H:
2,2-Dimethyl-4-(1H-4-pyridon-1-yl)-6-cyan-3-chromanol, F. 301°
2,2-Dimethyl-4-(2-hydroxy-4-pyridyloxy)-6-cyan-3-chromanol, F. 249-249,5° (aus Ethanol; daneben wenig 2,2-Dimethyl-4-(4-hydroxy-1H-2-pyridon-1-yl)-6-cyan-3-chromanol, F. 256-257°, aus Aceton)
2,2-Dimethyl-4-(2-hydroxy-4-pyridyloxy)-6-nitro-3-chromanol, F. 224-226°
2,2-Dimethyl-4-(2-hydroxy-4-pyridyloxy)-6-acetoxy-3-chromanol, F. 251-252°
2,2-Dimethyl-4-(2-hydroxy-4-pyridyloxy)-6-thiocarbamoyl-3-chromanol, F. 242°
2,2-Dimethyl-4-(1H-3,5-dichlor-4-pyridon-1-yl)-6-cyan-3-chromanol, F. > 250°

2,2-Dimethyl-4-(1H-2-Methoxy-4-pyridon-1-yl)-6-cyan-3-chromanol, F. 200-202°
2,2-Dimethyl-4-(1H-4-pyrimidinon-1-yl)-6-cyan-3-chromanol, F. 308-310°
2,2-Dimethyl-4-(2H-1-isochinolinon-2-yl)-6-cyan-3-chromanol, F. 135°
2,2-Dimethyl-4-(2H-1-phthalazinon-2-yl)-6-cyan-3-chromanol, F. 205°
2,2-Dimethyl-4-(1H-2-pyridon-1-yl)-6-(4-pyridyl)-3-chromanol, F. 216-218°
2,2,3-Trimethyl-4-(6-hydroxy-3-pyridazinyloxy)-6-cyan-3-chromanol, F. 240°.

Beispiel 3

Eine Lösung von 2,66 g 2,2-Dimethyl-4-brom-6-cyan-chroman (F. 89-92°; erhältlich durch Reduktion von 2,2-Dimethyl-6-cyan-4-chromanon mit $NaBH_4$ in $CH_3OH$ zu öligem 2,2-Dimethyl-6-cyan-4-chromanol und Umsetzung mit $PBr_3$ in Toluol bei 20°) und 2 g 1H-2-Pyridon in 70 ml Dimethylsulfoxid wird mit 1,2 g 80%igem NaH versetzt und 3 Tage bei 20° gerührt. Nach üblicher Aufarbeitung erhält man 2,2-Dimethyl-4-(1H-2-pyridon-1-yl)-6-cyan-chroman, F. 159°.

Analog erhält man mit den entsprechenden Verbindungen der Formel $R^5$-H:
2,2-Dimethyl-4-(1H-4-pyridon-1-yl)-6-cyan-chroman, F. 141-142° (mit 4-Hydroxypyridin = 1H-4-Pyridon);
2,2-Dimethyl-4-(1H-6-hydroxy-3-pyridazinyloxy)-6-cyan-chroman, F. 221-224° (mit 3,6-Pyridazindiol = 3-Hydroxy-1H-6-pyridazinon).

Beispiel 4

Ein Gemisch von 10 g "B", 3 g Natriumhydroxid und 350 ml Dioxan wird 20 Min. gekocht. Man kühlt ab, filtriert, dampft das Filtrat ein und erhält "A", F. 146-148°.

Analog erhält man durch Dehydratisierung der entsprechenden 3-Hydroxychromane:
2,2,3-Trimethyl-4-(1H-2-pyridon-1-yl)-6-cyan-2H-chromen, F. 212°
2,2-Dimethyl-4-(1H-4-pyridon-1-yl)-6-cyan-2H-chromen, F. 211°
2,2-Dimethyl-4-(1H-3,5-dichlor-4-pyridon-1-yl)-6-cyan-2H-chromen, F. 298-299°
2,2-Dimethyl-4-(2H-1-isochinolinon-2-yl)-6-cyan-2H-chromen, F. 171-172°
2,2-Dimethyl-4-(1H-2-pyridon-1-yl)-6-(4-pyridyl)-2H-chromen, F. 175-176°.

Beispiel 5

Zu einer Lösung von 3,13 g 2,2-Dimethyl-4-(1H-3-hydroxy-6-pyridazinon-1-yl)-6-cyan-chroman-3-ol in 160 ml DMF gibt man 9,5 g $K_2CO_3$ und 6 ml Dimethylsulfat, rührt 16 Std., gießt auf Eis, arbeitet wie üblich auf und erhält 2,2-Dimethyl-3-methoxy-4-(1H-3-methoxy-6-pyridazinon-1-yl)-6-cyan-chroman, F. 190-192°.

Analog erhält man
2,2-Dimethyl-3-methoxy-4-(1H-2-pyridon-1-yl)-6-cyan-chroman, F. 169-171°.

Beispiel 6

Zu einer Lösung von 298 mg 2,2-Dimethyl-4-(1H-2-pyridon-1-yl)-6-nitro-2H-chromen in 8 ml Dichlormethan tropft man bei 20° unter Rühren eine Lösung von 0,5 g 3-Chlorperbenzoesäure in 5 ml Dichlormethan, rührt 16 Std. bei 20°, gibt weitere 0,5 g 3-Chlorperbenzoesäure in 5 ml Dichlormethan hinzu, rührt noch 2 Tage, filtriert, arbeitet das Filtrat wie üblich auf und erhält 2,2-Dimethyl-3,4-epoxy-4-(1H-2-pyridon-1-yl)-6-nitrochroman, F. 130-132°.

Analog erhält man durch Epoxidierung der entsprechenden Chromene:
2,2-Dimethyl-3,4-epoxy-4-(1H-2-pyridon-1-yl)-6-cyan-chroman, F. 130-131°
2,2-Dimethyl-3,4-epoxy-4-(1H-2-pyridon-1-yl)-6-carbamoyl-chroman, F. 162-164°
2,2-Dimethyl-3,4-epoxy-4-(1H-2-pyridon-1-yl)-6-acetoxy-chroman.

Beispiel 7

Man leitet 45 Min. HCl in eine siedende Lösung von 12,5 g 2,2-Dimethyl-3,4-epoxy-4-(1H-2-pyridon-1-yl)-6-cyan-chroman in 100 ml Dioxan, kühlt ab, filtriert das ausgefallene 2,2-Dimethyl-4-(1H-2-pyridon-1-yl)-6-cyan-3-chromanon ab und wäscht mit Dioxan, dann mit Ether. F. 188-190°.

Analog erhält man durch Isomerisierung der entsprechenden Epoxide:

2,2-Dimethyl-4-(1H-2-pyridon-1-yl)-6-nitro-3-chromanon 2,2-Dimethyl-4-(1H-2-pyridon-1-yl)-6-carbamoyl-3-chromanon

2,2-Dimethyl-4-(1H-2-pyridon-1-yl)-6-acetoxy-3-chromanon, F. 137-139°.

Beispiel 8

Ein Gemisch von 2 g "B", 11,7 ml Ameisensäure und 3,3 ml Acetanhydrid wird 16 Std. bei 20° stehengelassen und anschließend 2 Std. auf 40-42° erwärmt. Nach Eindampfen und üblicher Aufarbeitung erhält man 2,2-Dimethyl-3-formyloxy-4-(1H-2-pyridon-1-yl)-6-cyan-chroman, F. 203,5-204°.

Beispiel 9

Ein Gemisch von 1 g "B" und 5 ml Acetanhydrid wird 1 Std. gekocht. Man kühlt ab, arbeitet wie üblich auf und erhält 2,2-Dimethyl-3-acetoxy-4-(1H-2-pyridon-1-yl)-6-cyan-chroman, F. 228-228,5°.

Beispiel 10

Man suspendiert 2,96 g "B" in 100 ml Wasser und tropft unter Rühren bei 10-20° 3,2 g Brom hinzu. Die Substanz löst sich, 2,2-Dimethyl-4-(1H-3,5-dibrom-2-pyridon-1-yl)-6-cyan-chroman-3-ol fällt aus und wird abfiltriert, F. 207-209°.

Beispiel 11

Man löst 2,78 g "A" in einem Gemisch von 10 ml konzentrierter Salpetersäure (68 %ig; D. 1,41) und 12 ml konzentrierter Schwefelsäure, rührt 3 Std. bei 20 °, gießt auf Eis, filtriert, wäscht mit Wasser und erhält ein Gemisch von 2,2-Dimethyl-4-(1H-3- und -5-nitro-pyridon-1-yl)-6-cyan-2H-chromen, das chromatographisch getrennt werden kann.

Beispiel 12

Eine Lösung von 1 g 2,2,3-Trimethyl-4-(1H-3-nitro-2-pyridon-1-yl)-6-methoxycarbonyl-chroman-3-ol in 25 ml Methanol wird bei 20° und 1 bar an 0,5 g 5 %igem Pd-C bis zum Stillstand hydriert. Man filtriert, dampft ein und erhält 2,2,3-Trimethyl-4-(1H-3-amino-2-pyridon-1-yl)-6-methoxycarbonyl-chroman-3-ol.

Beispiel 13

Eine Lösung von 1 g 2,2,3-Trimethyl-4-(1H-3-amino-2-pyridon-1-yl)-6-cyan-2H-chromen in 15 ml HCOOH und 1 ml Pyridin wird 19 Std. gekocht und eingedampft. Nach üblicher Aufarbeitung erhält man 2,2,3-Trimethyl-4-(1H-3-formamido-2-pyridon-1-yl)-6-cyan-2H-chromen.

Beispiel 14

Ein Gemisch von l g 2,2,3-Trimethyl-4-(1H-5-amino-2-pyridon-1-yl)-6-cyan-2H-chromen, 10 ml Acetanhydrid und 10 ml Pyridin wird 16 Std. bei 20° stehengelassen. Man dampft ein, reinigt chromatographisch und erhält 2,2,3-Trimethyl-4-(1H-5-acetamido-2-pyridon-1-yl)-6-cyan-2H-chromen.

Beispiel 15

In eine siedende Lösung von 1 g "A" in 50 ml Methanol und 2 ml Wasser wird 14 Std. unter Rühren HCl eingeleitet. Man läßt erkalten und über Nacht stehen. Die ausgefallene 2,2-Dimethyl-4-(1H-2-pyridon-1-yl)-2H-chromen-6-carbonsäure wird abfiltriert, F. 281-284°.

Beispiel 16

Ein Gemisch von 2,78 g "A", 31 g $Na_3PO_4$.12 $H_2O$, 28 ml Pyridin, 28 ml Wasser, 67 ml Essigsäure und 25 g Raney-Ni (wasserfeucht) wird bei 20° 3 Std. gerührt. Nach Filtration arbeitet man wie üblich auf und erhält 2,2-Dimethyl-4-(1H-2-pyridon-1-yl)-6-formyl-2H-chromen, F. 160-162°.

Beispiel 17

Man löst 2,78 g "A" in 40 ml tert.-Butanol und gibt unter Rühren 5,6 g gepulvertes KOH hinzu. Nach 1 Std. Kochen und üblicher Aufarbeitung erhält man 2,2-Dimethyl-4-(1H-2-pyridon-1-yl)-6-carbamoyl-2H-chromen.

Beispiel 18

In eine Lösung von 2,78 g "A" in einem Gemisch von 20 ml Pyridin und 10 ml Triethylamin leitet man 5 Std. bei 20° $H_2S$ ein, dampft ein, arbeitet wie üblich auf und erhält 2,2-Dimethyl-4-(1H-2-pyridon-1-yl)-6-thiocarbamoyl-2H-chromen, F. 254-257°.

Beispiel 19

Ein Gemisch von 310 mg "B", 808 mg Lawesson-Reagenz und 50 ml Toluol wird 1 Std. unter $N_2$ gekocht. Übliche Aufarbeitung gibt 2,2-Dimethyl-4-(1H-2-thiopyridon-1-yl)-6-cyan-chroman-3-ol.

Analog erhält man aus "A" das 2,2-Dimethyl-4-(1H-2-thiopyridon-1-yl)-6-cyan-2H-chromen.

Die nachstehenden Beispiele betreffen pharmazeutische Zubereitungen, die Verbindungen der Formel I oder ihre physiologisch unbedenklichen Salze enthalten:

Beispiel A Tabletten

Ein Gemisch von 1 kg 2,2-Dimethyl-4-(1H-2-pyridon-1-yl)-6-cyan-2H-chromen ("A"), 400 kg Lactose, 120 kg Kartoffelstärke, 20 kg Talk und 10 kg Magnesiumstearat wird in üblicher Weise zu Tabletten gepreßt, derart, daß jede Tablette 0,1 mg Wirkstoff enthält.

Beispiel B Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C Kapseln

Man füllt 1 kg 2,2-Dimethyl-4-(6-hydroxy-3-pyridazinyloxy)-6-cyan-3-chromanol ("B") in üblicher Weise in Hartgelatinekapseln, so daß jede Kapsel 0,5 mg Wirkstoff enthält.

Beispiel D Ampullen

Eine Lösung von 100 g 2,2-Dimethyl-4-(6-hydroxy-3-pyridazinyloxy)-6-cyan-3-chromanol in 2000 l zweifach destilliertem Wasser wird steril filtriert und in Ampullen abgefüllt. Jede Ampulle enthält 0,1 mg

Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

Beispiel E Lösung zur topischen Anwendung (gegen Alopezie)

Man löst 500 g "A" (oder "B") in einem Gemisch von 5,2 kg 1,2-Propandiol und 15 l Ethanol, füllt mit Ethanol auf 25 l auf, filtriert steril und füllt in Flaschen.

Beispiel F Gel

Man mischt 0,45 g Carbopol 934 P (= Carboxyvinyl-polymer) mit 40 ml zweifach destilliertem Wasser und 27 ml Ethanol, gibt eine Lösung von 0,5 g "A" (oder "B") und 0,45 g Diisopropanolamin in 10 ml 1,2-Propandiol und 13 ml Ethanol hinzu, mischt gut durch, füllt mit Wasser auf 100 ml auf, und mischt erneut gut durch. Das erhaltene Gel enthält 0,5 Gew.% Wirkstoff.

## Ansprüche

1. Chromanderivate der Formel I

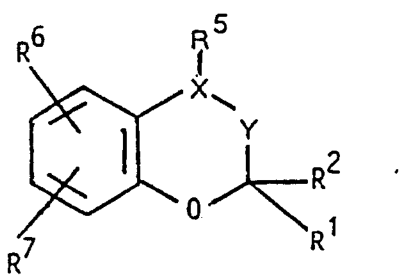

I

worin

X-Y

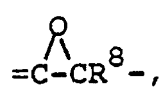

=CH-CO- oder =CH-CHR$^8$-, sowie, falls der Rest R$^5$ weder vollständig noch partiell hydriert ist, auch =CR$^4$-CR$^3$R$^8$-oder =CH-CR$^8$(OA)-,

R$^1$ A,

R$^2$ und R$^8$ jeweils H oder A,

R$^1$ und R$^2$ zusammen auch Alkylen mit 3-6 C-Atomen,

R$^3$ OH oder OAc,

R$^4$ H,

R$^3$ und R$^4$ zusammen auch eine Bindung,

R$^5$ einen unsubstituierten oder einen ein-oder zweifach durch A, F, Cl, Br, J, OH, OA, OAc, $NO_2$, $NH_2$ AcNH, HOOC und/oder AOOC substituierten Pyridyl-oxy-, Pyridazinyl-oxy-, Pyrimidinyl-oxy-, Pyrazinyl-oxy-, Oxo-dihydro-pyridyl-oxy-, Oxo-dihydro-pyridazinyl-oxy-, Oxo-dihydro-pyrimidinyl-oxy-, Oxo-dihydro-pyrazinyl-oxy-, 1H-2- oder 1H-4-Pyridon-1-yl-, 1H-4- oder 1H-6-Pyridazinon-1-yl-, 1H-2-, 1H-4- oder 1H-6-Pyrimidinon-1-yl-, 1H-2-Pyrazinon-1-yl-, 3H- oder 5H-2-Pyrrolinon-1-yl-, 1H-2-Chinolinon-1-yl-, 2H-1-Isochinolinon-2-yl-, 2H-1-Phthalazinon-2-yl-, 3H-4-Chinazolinon-3-yl- oder 1H-2-Thiopyridon-1-yl-rest, wobei diese Reste auch vollständig oder partiell hydriert sein können,

R$^6$ und R$^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J,

$CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$ $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyanalkyl, A-C(=NOH) oder A-C(=$NH_2$),
$R^7$ auch Pyridyl, Pyridazinyl, Pyrimidinyl oder Pyrazinyl,
A Alkyl mit 1-6 C-Atomen,
alkyl Alkylen mit 1-6 C-Atomen und
Ac Alkanoyl mit 1-8 C-Atomen oder Aroyl mit 7-11 C-Atomen
bedeuten,
sowie deren Salze.

2. Verbindungen der Formel I gemäß Patentanspruch 1, worin

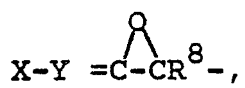

=CH-CO- oder =CH-CH$R^8$, sowie, falls $R^5$ einen unsubstituierten oder einen ein- oder zweifach durch A, F, Cl, Br, J, OH, OA, OAc, $NO_2$, $NH_2$, AcNH, HOOC und/oder AOOC substituierten 1H-4-Pyridon-1-yl-, 1H-4-Pyridazinon-1-yl-, 1H-4-Pyrimidinon-1-yl-, 1H-2-Chinolinon-1-yl-, 2H-1-Isochinolinon-2-yl-, 2H-1-Phthalazinon-2-yl- oder 3H-4-Chinazolinon-3-yl-Rest (wobei diese Reste auch vollständig oder partiell hydriert sein können) und/oder falls $R^7$ Pyridyl, Pyridazinyl, Pyrimidinyl oder Pyrazinyl bedeutet, auch =C$R^4$-C$R^3R^8$-, oder, falls der Rest $R^5$ weder vollständig noch partiell hydriert ist, auch =CH-C$R^8$(OA)- bedeutet.

3.
a) 2,2-Dimethyl-4-(1H-2-pyridon-1-yl)-6-cyan-chroman-3-ol;
b) 2,2-Dimethyl-4-(1H-2-pyridon-1-yl)-6-cyan-2H-chromen;
c) 2,2-Dimethyl-4-(6-hydroxy-3-pyridazinyloxy)-6-cyan-3-chromanol.

4. Verfahren zur Herstellung von Chromanderivaten der Formel I, dadurch gekennzeichnet, daß man zur Herstellung von Verbindungen der Formel I, worin X-Y =C$R^4$-C$R^3R^8$ oder =CH-CH$R^8$ bedeutet, ein 3,4-Epoxychroman der Formel IIa oder ein Chromanderivat der Formel IIb

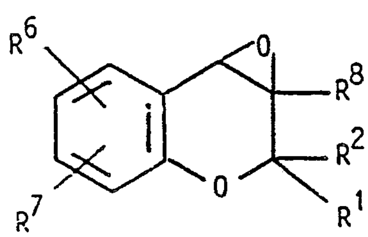

IIa

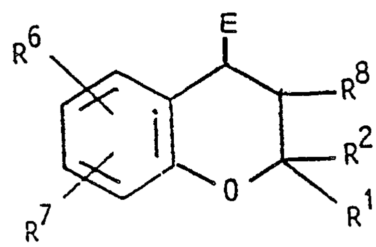

IIb

worin
E Cl, Br, J oder eine reaktionsfähig veresterte OH-Gruppe bedeutet und
$R^1$, $R^2$, $R^6$, $R^7$ und $R^8$ die bei Formel I angegebene Bedeutung haben,
mit einer Verbindung der Formel III
$R^5$-H III
worin $R^5$ die bei Formel I angegebene Bedeutung hat oder mit einem ihrer reaktionsfähigen Derivate umsetzt
und/oder daß man eine Verbindung der Formel I, worin X-Y =CH-C$R^8$(OH)- bedeutet, dehydratisiert oder alkyliert und/oder daß man eine Verbindung der Formel I, worin X-Y =C=C$R^8$- bedeutet, epoxidiert und/oder daß man eine Verbindung der Formel I, worin

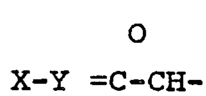

bedeutet, durch Behandeln mit einem sauren Katalysator zu einer Verbindung der Formel I, worin X-Y =CH-CO- bedeutet, isomerisiert und/oder daß man in einer Verbindung der Formel I einen oder mehrere der Reste $R^3$, $R^5$, $R^6$ und /oder $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

5. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfstoff und gegebenenfalls in Kombination mit einem oder mehreren weiteren Wirkstoff(en) in eine geeignete Dosierungsform bringt.

6. Pharmazeutische Zubereitung, gekennzeichnet durch einen Gehalt an mindestens einer Verbindung der Formel I und/oder einem ihrer physiologisch unbedenklichen Salze.

7. Verwendung von Verbindungen der Formel I zur Herstellung eines Arzneimittels.

PAT LOG 5-A 110489

8. Verwendung von Verbindungen der Formel I zur Herstellung eines Arzneimittels zur Behandlung der Alopezie.

9. Verwendung von Verbindungen der Formel I bei der Bekämpfung von Krankheiten.

Europäisches Patentamt

# EUROPÄISCHER RECHERCHENBERICHT

Nummer der Anmeldung

EP 89 11 0177

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 076 075 (BEECHAM GROUP PLC) * Seite 1, Anspruch 1; Seite 5, Anspruch 15; Seite 7, Anspruch 20 * | 1,2,4,5 | C 07 D 405/04<br>C 07 D 405/12<br>A 61 K 31/44 //<br>(C 07 D 405/04<br>C 07 D 311:00<br>C 07 D 211:00 )<br>(C 07 D 405/04<br>C 07 D 311:00<br>C 07 D 237:00 )<br>(C 07 D 405/04<br>C 07 D 311:00<br>C 07 D 217:00 )<br>(C 07 D 405/12<br>C 07 D 311:00<br>C 07 D 211:00 )<br>(C 07 D 405/12<br>C 07 D 311:00<br>-/- |
| A | EP-A-0 107 423 (BEECHAM GROUP PLC) * Seiten 22,23,29; Beispiele 5,6,12; Seite 5, Anspruch 8, Seite 7, Anspruch 10 * | 1,2,4,5 | |
| A | JOURNAL OF MEDICINAL CHEMISTRY Band 29, Nr. 11, 1986, Seiten 2194-2201; J. M. EVANS et al.: "Synthesis and Antihypertensive Activity of 4-(Cyclic amido)-2H-1-benzopyrans" * Seite 2195, Tabelle I, Verbindungen 52-56; Seite 2197, Tabellen III,IV * | 1,2,4,5 | |
| A | JOURNAL OF THE CHEMICAL SOCIETY, PERKIN TRANSACTIONS I nr. 12, 1983, Seiten 2903-2912; B. R. BROWN et al.: "Synthesis and Reactions of 4-Aryloxyflavans" * Seite 2905, Verbindung 9 * | 1 | **RECHERCHIERTE SACHGEBIETE (Int. Cl.4)** |
| A | CHEMICAL ABSTRACTS Band 84, Nr. 5, 2. Februar 1976, Seite 451, Spalte 1, Zusammenfassung Nr. 30936m, Columbus, Ohio, USA; S. M. EL-ANTABLY et al.: "Synthesis and blood pressure lowering activity of benzylic ethers of 2-diethylaminoethanol and a related diamine", & J. Pharm. Sci. Band 64, Nr. 8, 1975, Seiten 1423-1425 -/- | 1,5 | C 07 D 311/00<br>C 07 D 405/00 |

Der vorliegende Recherchenbericht wurde für alle Patentansprüche erstellt

| Recherchenort | Abschlußdatum der Recherche | Prüfer |
|---|---|---|
| BERLIN | 01-08-1989 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 11 0177

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 273 262 (MERCK PATENT GMBH) * Seite 16, Anspruch 1; Seite 17, Ansprüche 2-4 * | 1,2,4,5 | C 07 D 217:00 ) |
| P,A | GB-A-2 204 868 (SANDOZ LTD.) * Seite 32, Anspruch 1; Seite 41, Anspruch 7; Seiten 42,43, Anspruch 9 * | 1,2,4,5 | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| BERLIN | 01-08-1989 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)